# EUROPEAN PATENT APPLICATION

(11) **EP 4 011 384 A1**
(43) Date of publication of application: **15.06.2022**
(21) Application number: 21179891.3
(22) Date of filing: 16.06.2021
(51) Int. Cl.: A61K 35/741, A61P 1/00, A61P 31/04

(54) **DECOLONIZATION OF ENTEROBACTERIA, SUCH AS KLEBSIELLA PNEUMONIAE, FROM THE GUT USING STRAINS OF KLEBSIELLA OXYTOCA**

(30) Priority: 09.12.2020 EP 20212877
(71) Applicant: Helmholtz-Zentrum für Infektionsforschung GmbH, 38124 Braunschweig (DE); Otto-von-Guericke-Universität Magdeburg, 39120 Magdeburg (DE)
(72) Inventor: Strowig, Till, 38124 Braunschweig (DE); Osbelt, Lisa, 38124 Braunschweig (DE); Wende, Marie, 38124 Braunschweig (DE)
(74) Representative: Krauss, Jan

(57) **Abstract**

The present invention relates to probiotic bacteria of the species *Klebsiella oxytoca* that are used for a decolonization of multidrug resistant (MDR) *Enterobacteria,* such as *Klebsiella pneumoniae (K. pneumoniae),* from the gut of a subject. The decolonization can both be therapeutic, i.e. after colonization of the gut by the multiresistant pathogen(s), or as a preventive measure before a re-colonization of the gut, as required after antibiotic treatment.

## Description

The present invention relates to probiotic bacteria of the species *Klebsiella oxytoca* that are used for a decolonization of multidrug resistant (MDR) *Enterobacteria,* such as *Klebsiella pneumoniae (K. pneumoniae),* from the gut of a subject. The decolonization can both be therapeutic, i.e. after colonization of the gut by the multiresistant pathogen(s), or as a preventive measure before a re-colonization of the gut, as required after antibiotic treatment.

### Background of the invention

One of the critical functions of a healthy intestinal microbiota is to provide colonization resistance against a variety of bona fide and potential pathogens. Anaerobic bacteria in the *Firmicutes* and *Bacteroidetes* phyla are the dominant constituents of the normal colonic microbiota and together can provide colonization resistance through both direct (bacteria-bacteria) and indirect (host mediated) mechanisms. Several mechanisms of colonization resistance against intestinal pathogens have been described, including competition for nutrients, short chain fatty acid (SCFA)-dependent inhibition of virulence and replication, and direct antagonism through bacteriocin production or Type VI Secretion System-mediated killing.

In healthcare settings, antibiotic-mediated disruption of the microbiota can be associated with enteric expansion of members of the *Enterobacteriaceae* family, including *E. coli* and *Klebsiella pneumoniae,* as well as vancomycin-resistant *Enterococci* and *Clostridium difficile* (Kim S., Covington A., and Pamer E.G. 2017. The intestinal microbiota: Antibiotics, colonization resistance, and enteric pathogens. Immunol. Rev. 279:90-105. 10.1111/imr.12563). While *C. difficile* causes gastroenteritis, hospital-associated *Enterobacteriaceae* and vancomycin-resistant *Enterococci* strains often expand in the gut without triggering overt inflammatory responses. In these instances, the primary clinical concern is that expansion of these species in the gut increases the risk for subsequent development of a bloodstream infection (BSI) in vulnerable patient populations. For example, allogeneic hematopoietic stem cell transplantation (allo-HCT) is an effective but highly immunocompromising treatment for some forms of cancer. The high incidence and risk of bacterial infections in patients undergoing allo-HCT necessitates administration of prophylactic and empiric antibiotics, leading to destruction of the normal microbiome. In this patient population, enteric domination with *Enterobacteriaceae* leads to a significant increase in the risk of developing a BSI. Furthermore, an increasing proportion of clinical isolates of *Enterobacteriaceae* are resistant to a wide range of antibiotics, including strains that produce extended-spectrum-β-lactamases or carbapenemases. Accordingly, BSI with these highly antibiotic-resistant strains are increasingly challenging to treat. *Klebsiella pneumoniae, Escherichia coli,* and other members of the *Enterobacteriaceae* family are common human pathogens that have acquired broad antibiotic resistance, rendering infection by some strains virtually untreatable. *Enterobacteriaceae* are intestinal residents, but generally represent <1% of the adult colonic microbiota.

Sorbara et al. (in: Sorbara MT, et al. Inhibiting antibiotic-resistant Enterobacteriaceae by microbiota-mediated intracellular acidification. J Exp Med. 2019 Jan 7;216(1):84-98. doi: 10.1084/jem.20181639. Epub 2018 Dec 18) demonstrate that an antibiotic-naive microbiota suppresses growth of antibiotic-resistant clinical isolates of *Klebsiella pneumoniae, Escherichia coli,* and *Proteus mirabilis* by acidifying the proximal colon and triggering short chain fatty acid (SCFA)-mediated intracellular acidification. High concentrations of SCFAs and the acidic environment counter the competitive edge that O₂ and NO₃ respiration confer upon *Enterobacteriaceae* during expansion. Reestablishment of a microbiota that produces SCFAs enhances clearance of *Klebsiella pneumoniae, Escherichia coli,* and *Proteus mirabilis* from the intestinal lumen and represents a potential therapeutic approach to enhance clearance of antibiotic-resistant pathogens.

Fecal microbiota transplant (FMT), also known as a stool transplant, is the process of transferring fecal bacteria and other microbes from a healthy individual into another individual. FMT involves restoration of the colonic microflora by introducing healthy bacterial flora through infusion of stool via colonoscopy, enema, orogastric tube, or by mouth in the form of a capsule containing feces from a healthy donor, which in some cases is freeze-dried. FMT is an effective treatment for *Clostridioides difficile* infection (CDI), and may be more effective than vancomycin treatment. Side effects may include a risk of infections, therefore the donor should be screened.

With CDI becoming more common, FMT is gaining increasing prominence, with some experts calling for it to become the first-line therapy for CDI. FMT has been used experimentally to treat other gastrointestinal diseases, including colitis, constipation, irritable bowel syndrome, and neurological conditions, such as multiple sclerosis and Parkinson's. In the United States, human feces has been regulated as an experimental drug since 2013.

Caballero et al,, (in: Silvia Caballero, et al., Cooperating Commensals Restore Colonization Resistance to Vancomycin-Resistant Enterococcus faecium, Cell Host & Microbe, Vol 21, 5, 2017, pp. 592-602.e4, https://doi.org/10.1016/j.chom.2017.04.002.2017) demonstrate that a precisely defined consortium of commensal bacteria containing the *Clostridium* cluster XIVa species *Blautia producta* and *Clostridium bolteae* restores colonization resistance against VRE and clears VRE from the intestines of mice.

O. Ljungquist et al. (in: Probiotics for intestinal decolonization of ESBL-producing Enterobacteriaceae: a randomized, placebo-controlled clinical trial. Clinical Microbiology and Infection 26 (2020) 456e462 https://doi.org/10.1016/j.cmi.2019.08.019) disclose a randomized, placebo-controlled, single-blinded clinical superiority trial, where the probiotic Vivomixx^{®}, a mixture of 8 different living bacterial strains or placebo was given to adult outpatients intestinally colonized for at least 3 months with extended spectrum b-lactamase (ESBL)-producing *Enterobacteriaceae* (EPE).

Lagrafeuille et a. (in: Lagrafeuille R, et al. Opposing effect of Lactobacillus on in vitro Klebsiella pneumoniae in biofilm and in an in vivo intestinal colonisation model. Benef Microbes. 2018 Jan 29;9(1):87-100. doi: 10.3920/BM2017.0002. Epub 2017 Oct 12. PMID: 29022382) analyze the anti-biofilm activity of 140 neutralized *Lactobacillus* supernatants was assessed against *K. pneumoniae.* Among the 13 strains whose supernatant significantly impaired biofilm formation, *Lactobacillus plantarum* CIRM653 was selected because it was also able to impair K. pneumoniae preformed biofilm, independently of a bactericidal effect.

Multi-drug resistance in the family of *Enterobacteriaceae* is one of the greatest health associated problem worldwide (WHO. United Nations meeting on antimicrobial resistance. Bull World Health Organ. 2016;94(9):638-639. doi:10.2471/blt.16.020916). Especially the incidence of infections as well as the mortality rates linked to carbapenem-resistant *K*. *pneumoniae* is constantly increasing and has reached alarming rates in Europe (Cassini, Alessandro et al (2018). Attributable deaths and disability-adjusted life-years caused by infections with antibiotic-resistant bacteria in the EU and the European Economic Area in 2015: a population-level modelling analysis. The Lancet Infectious Diseases. 19. 10.1016/S1473-3099(18)30605-4). The search for alternative intervention strategies new therapeutics is therefore urgently required. Other objects and advantages will become apparent to the person of skill when studying the present description of the present invention.

In a first aspect of the present invention, the above object is solved by a bacterium selected from the group of *Klebsiella oxytoca* and species related to *Klebsiella oxytoca* for use in the prevention and/or treatment of antibiotic resistant *Enterobacteriaceae,* such as *Klebsiella pneumoniae,* in particular multi-resistant *Enterobacteriaceae,* in the microbiome of an animal, in particular a mammalian subject, wherein said bacterium selected from the group of *Klebsiella oxytoca* and species related to *Klebsiella oxytoca* outcompetes said *Enterobacteriaceae* for use of beta-glucosidic sugars such as sucrose and/or cellobiose. Preferred is the *Klebsiella oxytoca* for use according to the present invention, wherein said prevention and/or treatment is through decolonization of said *Enterobacteriaceae.*

In a second aspect of the present invention, the above object is solved by the bacterium for use as above in reestablishing colonization resistance after antibiotic dysbiosis in the microbiome of an animal, in particular a mammalian subject.

A third aspect of the present invention then relates to a method for preventing and/or treating antibiotic resistant *Enterobacteriaceae,* such as *Klebsiella pneumoniae,* in the microbiome of an animal, in particular a mammalian subject, such as a human, comprising administering to said animal, in particular said mammalian subject an effective amount of a bacterium selected from the group of *Klebsiella oxytoca* and species related to *Klebsiella oxytoca,* wherein said bacterium selected from the group of *Klebsiella oxytoca* and species related to *Klebsiella oxytoca* outcompetes said *Enterobacteriaceae* for use of beta-glucosidic sugars such as sucrose and/or cellobiose, and wherein preferably said related species is selected from the group consisting of *K. michiganensis, K. grimontii,* K. *aerogenes,* and *Klebsiella* that have a carbohydrate utilization pattern substantially similar to *K. oxytoca* strain MK01.

It was surprisingly found that isolates of the bacterium *Klebsiella oxytoca* and species related to *Klebsiella oxytoca* constitute an effective probiotic for the prevention and/or treatment of antibiotic resistant *Enterobacteriaceae,* such as *Klebsiella pneumoniae,* in the microbiome of an animal, in particular a mammalian subject. Apparently, *Klebsiella oxytoca* and species related to *Klebsiella oxytoca* outcompete said *Enterobacteriaceae* for use of beta-glucosidic sugars such as sucrose and/or cellobiose, and leads to an effective decolonization of *Enterobacteriaceae,* which is particularly useful in case of multidrug resistant (MDR) *Enterobacteria,* such as *Klebsiella pneumoniae,* and thus causes a prevention and/or treatment of said pathogen(s). The decolonization can both be therapeutic, i.e. after colonization of the gut by the (multiresistant) pathogen(s), or as a preventive measure before a re-colonization of the gut, as required after antibiotic treatment (see also Examples, below). The prevention and/or treatment of antibiotic resistant *Enterobacteriaceae,* such as *Klebsiella pneumoniae,* in the microbiome of an animal, in particular a mammalian subject can be preferably embodied as a long-lasting effect in combination with at least one bacterial species selected from the group consisting of Firmicutes spec., and preferably *Blautia coccoides* YL58, *Enterococcus faecalis* KB1 and *Clostridium clostridioforme* YL32, and in particular human isolates thereof.

Preferred is therefore the bacterium for use according to the present invention, wherein said prevention and/or treatment is through decolonization of said *Enterobacteriaceae.*

In the context of the present invention, the term *"Klebsiella oxytoca",* in particular in the context with the phrase "bacterium for use" as herein, shall generally relate to the species of the Gram-negative bacterium including species related to *Klebsiella oxytoca,* and includes individual strains or isolates or mixtures thereof. The term includes *Klebsiella oxytoca* and species related to *Klebsiella oxytoca* that outcompete the *Enterobacteriaceae* for the metabolic use of beta-glucosidic sugars such as sucrose and/or cellobiose. The term also includes commensal *Klebsiella* strains, *K. michiganensis, K. grimontii,* K. *aerogenes,* and *Klebsiella* that have a carbohydrate utilization pattern substantially similar to *K. oxytoca* strain MK01, and in particular human commensals. *Klebsiella oxytoca* is naturally resistant to ampicillin, amoxicillin, ticarcillin and to antibiotics to which other *Enterobacteriaceae* are also intrinsically resistant (herein referred to as "naturally occurring ampicillin/amoxicillin-resistance phenotype"). Examples in the context of the present invention are the isolates *K. oxytoca* strain MK01 isolated from donor MK1903 *(K. oxytoca* MK01), and *K. oxytoca* strain MK02 isolated from donor MK1901 (*K. oxytoca* MK02) (see Examples). Further preferred is a *Klebsiella oxytoca* for use according to the present invention, wherein said *Klebsiella oxytoca* for use is a strain or an isolate isolated from a human.

Even further preferred are *Klebsiella oxytoca* for use according to the present invention, wherein said *Klebsiella oxytoca* does not exhibit additional antibiotic-resistance in addition to the naturally occurring ampicillin/amoxicillin-resistance phenotype. These bacteria were found to provide an effective therapeutic and/or preventive, and at the same time this strategy further reduces the risk of spreading antibiotic resistance markers in the bacterial community.

Antibiotic resistant *Enterobacteriaceae,* such as *Klebsiella pneumoniae,* in the context of the present invention, shall mean any species or strain (or mixtures thereof) of *Enterobacteriaceae* that has acquired or has acquired an additional resistance phenotype, compared to a sensitive strain (disregarding the naturally occurring resistance phenotypes). Such antibiotic resistance is more available than ever before to organisms such as *Escherichia coli* and *Klebsiella pneumoniae* that are important causes of major sepsis (see, for example, Iredell Jon, Brown Jeremy, Tagg Kaitlin. Antibiotic resistance in *Enterobacteriaceae:* mechanisms and clinical implications BMJ 2016; 352 :h6420). Examples are resistant strains belonging to *Citrobacter, Enterobacter, Hafnia, Klebsiella, Proteus, Providencia, Salmonella,* and *Yersinia.* Additional examples of antibiotic resistant *Enterobacteriaceae* are multidrug resistant *Enterobacteriaceae* (MDR), a concerning problem in clinical environments in both the hospital and the ambulatory setting. Carbapenem-resistant *Enterobacteriaceae* are regarded as an urgent threat by the CDC in the US.

Preferred is therefore the bacterium for use according to the present invention, wherein said *Enterobacteriaceae* is/are multi-resistant *Enterobacteriaceae.*

In the context of the present invention, the term "microbiome" shall refer to the microbial community occupying the intestines of a mammalian subject, such as a human. A further example is the Oligo-Mouse-Microbiota (Oligo-MM¹²), a community of 12 mouse intestinal bacteria to be used for microbiome research in gnotobiotic mice, consisting of *Clostridium innocuum, Bacteroides caecimuris, Lactobacillus reuteri, Enterococcus faecalis, Acutalibacter muris, Bifidobacterium animalis* subsp. *animalis, Muribaculum intestinale, Flavonifractor plautii, Clostridium clostridioforme, Akkermansia muciniphila, Turicimonas muris,* and *Blautia coccoides* (Garzetti D, Brugiroux S, Bunk B, et al. High-Quality Whole-Genome Sequences of the Oligo-Mouse-Microbiota Bacterial Community. Genome Announc. 2017;5(42):e00758-17. Published 2017 Oct 19. doi:10.1128/genomeA.00758-17). Preferred is the *Klebsiella oxytoca* for use according to the present invention, wherein said microbiome is located in the gut of said animal, in particular a mammalian subject, and wherein said subject is selected from poultry, such as chicken or geese, a mouse, rat, cat, dog, rabbit, goat, sheep, horse, camel, lama, cow, monkey, a farm animal, a sport animal, and a pet, and a human.

The intestinal microbiota plays beneficial roles in many physiological processes of the host. Dysbiosis, a disruption of microbial composition by various stresses, has been implicated in inflammatory bowel disease (IBD), colon cancer, obesity, asthma, and other diseases. In particular, excessive dosing of antibiotics elicits the loss of naturally occurring intestinal microbiota. Such loss increases the numbers of yeasts, such as *Candida albicans,* and bacteria, such as *Proteus, Staphylococcus,* and *Clostridium difficile* (*C. difficile*), that normally exist at low numbers, leading to depression of digestive functions or the occurrence of intestine-related diseases. The idea that probiotics could improve or prevent diarrhea began with the notion that these gut-associated diseases were caused by the collapse of "colonization resistance" due to the absence of normal bacterial flora. Probiotics are known to be very effective for the treatment of antibiotic-associated diarrhea, with *Saccharomyces boulardii* (*S. boulardii*)*, E. coli* Nissle 1917, *Lactobacillus,* and *Bifidobacterium* as the main focus of research. Application of FMT and probiotics for eradication of gastrointestinal diseases and enteropathogens exhibits the potential to restore the degraded ecosystem and protection against colonization and proliferation of enteropathogens. Therefore, in another aspect of the present invention, the bacterium is used and/or is for use according to the present invention in reestablishing colonization resistance after antibiotic dysbiosis in the microbiome of a mammalian subject.

Another aspect of the present invention then relates to the bacterium for use according to the present invention, wherein said prevention and/or treatment of said antibiotic resistant *Enterobacteriaceae* and/or said reestablishing colonization resistance after antibiotic dysbiosis is in the context of bacterial infection, bloodstream infection, allogeneic hematopoietic stem cell transplantation, and bacterial invasion into the liver, spleen and/or mesenteric lymph nodes (MLN) in said animal, in particular mammalian subject. As further described below, luminal and tissue invasion of *K. pneumoniae* MDR1 was assessed in the gastrointestinal organs including small intestine, cecum and colon as well as in the liver, and lymphatic organs including spleen and mesenteric lymph nodes (MLN) on day 6 p.c. A strong reduction in the gastrointestinal organ content as well as in the tissues in both precolonized groups compared to the control groups was observed. In addition, *Klebsiella oxytoca* colonized groups did not have any bacteria in the liver, spleen or MLN in contrast to the control animals, suggesting a systemic spread of the pathogen in control mice, elevating the risk for blood stream infections. This experiment demonstrated a broad-spectrum activity of *Klebsiella oxytoca* in all gastrointestinal organs including reduction of pathogenic bacteria in the lumen and tissue and preventing the systemic spread into the liver and lymphatic tissues such as spleen and MLN.

The bacterium for use according to the present invention can be administered to the mammalian subject in any suitable way that allows for colonization, in particular a substantial colonization, with said bacterium, for example as a pharmaceutical composition comprising an effective amount of the *Klebsiella oxytoca* for use according to the present invention, and a pharmaceutically or therapeutically acceptable excipient or carrier. Compositions are for fecal microbiota transplant (FMT), comprise a suitable medium, such as sterile saline, are used as a suitable solid dosage form, such as a suppository or capsule, in particular stomach-acid resistant, and/or as a probiotic.

The term "pharmaceutically or therapeutically acceptable excipient or carrier" refers to a solid or liquid filler, diluent or encapsulating substance which does not interfere with the effectiveness or the biological activity of the active ingredients (here, bacteria) and which is not toxic to the host, which may be either humans or animals, to which it is administered. Depending upon the particular route of administration, a variety of pharmaceutically-acceptable carriers such as those well known in the art may be used. Non-limiting examples include sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water. Pharmaceutically acceptable carriers or excipients also include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/cosolvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for use according to the present invention, and will readily be able to choose suitable pharmaceutically acceptable carriers or excipients, depending, e.g., on the formulation and administration route of the pharmaceutical composition. In a preferred example, the *Klebsiella oxytoca* for use according to the present invention is provided as a probiotic, i.e. a live microbial food or feed supplement (preparation) which beneficially affects the host animal by improving its intestinal microbial balance.

In addition to the aforementioned bacteria for use according to the invention, the pharmaceutical composition as administered can contain other therapeutically active substances - for example with medicaments already known for the treatment of the aforementioned conditions and/or diseases, in particular suitable nutrients and supplements for bacterial growth as desired, e.g. butyrate, but also suitable antibiotics, whereby in the latter case a favorable additive, amplifying or preferably synergistically effect is noticed.

Also preferred is the *Klebsiella oxytoca* for use according to the present invention, wherein said use is in combination with at least one bacterial species selected from the group consisting of *Clostridium* spec., in particular *Clostridium* innocuum and *Clostridium clostridioforme. Bacteroides* spec., in particular *Bacteroides caecimuris, Lactobacillus* spec., in particular *Lactobacillus reuteri, Enterococcus* spec., in particular *Enterococcus faecalis, Acutalibacter* spec., in particular *Acutalibacter muris, Bifidobacterium* spec., in particular *Bifidobacterium animalis* subsp. *animalis, Muribaculum* spec., in particular *Muribaculum intestinale, Flavonifractor* spec., in particular *Flavonifractor plautii, Akkermansia* spec., in particular *Akkermansia muciniphila, Turicimonas* spec., in particular *Turicimonas muris,* and *Blautia* spec., in particular *Blautia coccoides,* Firmicutes spec., and preferably *Blautia coccoides* YL58, *Enterococcus faecalis* KB1 and *Clostridium clostridioforme* YL32, and in particular human isolates thereof, and optionally with at least one additional therapeutically active substance as above. As the protective effect of the bacterium for use according to the invention was also found in Oligo-MM¹² mice, there is the potential of each of the twelve bacteria to establish long term protection together with *Klebsiella oxytoca* by single transfer of bacteria into the subject to be treated.

The dosage of the pharmaceutical composition to be administered according to the present invention can be appropriately selected according to the route of administration, the subject to be administered, the target disease and its severity, age, sex weight, individual differences and disease state. Dosage may be repeated several times a day. Preferred is a bacterium for use according to the present invention, wherein said use is in a (single) dosage of between 10¹² and 10⁷ CFU, preferably between 10¹⁰ and 10⁸ CFU of said bacterium, e.g. *Klebsiella oxytoca.* Doses can be applied several times, as needed.

Yet another aspect of the present invention then relates to a method for preventing and/or treating antibiotic resistant *Enterobacteriaceae,* such as *Klebsiella pneumoniae,* in the microbiome of an animal, in particular a mammalian subject, such as a human, comprising administering to said animal, in particular mammalian subject an effective amount of a bacterium selected from the group of *Klebsiella oxytoca* and species related to *Klebsiella oxytoca,* wherein said bacterium selected from the group of *Klebsiella oxytoca* and species related to *Klebsiella oxytoca* outcompetes said *Enterobacteriaceae* for use of beta-glucosidic sugars such as sucrose and/or cellobiose, and wherein preferably said related species is selected from the group consisting of *K. michiganensis, K. grimontii,* K. *aerogenes,* and *Klebsiella* that have a carbohydrate utilization pattern substantially similar to *K. oxytoca* strain MK01.

By "treatment" or "treating" is meant any treatment of a disease or disorder, in a mammal, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes, but does not require complete recovery or complete prevention of a stress.

As mentioned above, in addition to the bacteria for use according to the invention, the pharmaceutical composition as administered can contain other therapeutically active substances - for example with medicaments already known for the treatment of the aforementioned conditions and/or diseases, in particular suitable nutrients, such as butyrate, and supplements for bacterial growth as desired, but also suitable antibiotics, whereby in the latter case a favorable additive, amplifying or preferably synergistically effect is noticed. Also preferred is method as above, wherein said prevention and/or treatment is in combination with at least one bacterial species selected from the group consisting of *Clostridium* spec., in particular *Clostridium* innocuum and *Clostridium clostridioforme. Bacteroides* spec., in particular *Bacteroides caecimuris, Lactobacillus* spec., in particular *Lactobacillus reuteri, Enterococcus* spec., in particular *Enterococcus faecalis, Acutalibacter* spec., in particular *Acutalibacter muris, Bifidobacterium* spec., in particular *Bifidobacterium animalis* subsp. *animalis, Muribaculum* spec., in particular *Muribaculum intestinale, Flavonifractor* spec., in particular *Flavonifractor plautii, Akkermansia* spec., in particular *Akkermansia muciniphila, Turicimonas* spec., in particular *Turicimonas muris,* and *Blautia* spec., in particular *Blautia coccoides,* Firmicutes spec., and preferably *Blautia coccoides* YL58, *Enterococcus faecalis* KB1 and *Clostridium clostridioforme* YL32, and in particular human isolates thereof, and optionally with at least one additional therapeutically active substance as above. As the protective effect of the *Klebsiella oxytoca* for use according to the invention was also found in Oligo-MM¹² mice, there is the potential of each of the twelve bacteria, in particular related human isolates thereof, to establish long term protection together with *Klebsiella oxytoca* by single transfer of bacteria into the subject to be treated.

It is to be understood that the present bacteria and/or a pharmaceutical composition comprising the present bacteria is for use to be administered to an animal, such as a mammalian, e.g. a human patient. The term "administering" means administration of a sole therapeutic bacterial agent or in combination with another therapeutic agent. It is thus envisaged that the pharmaceutical composition of the present invention are employed in co-therapy approaches, i.e. in co-administration with other medicaments or drugs and/or any other therapeutic agent which might be beneficial in the context of the methods of the present invention. Nevertheless, the other medicaments or drugs and/or any other therapeutic agent can be administered separately from the compound for use, if required, as long as they act in combination (i.e. directly and/or indirectly, preferably synergistically) with the present compound(s) (for use).

The dosage of the pharmaceutical composition to be administered in the method according to the present invention can be appropriately selected according to the route of administration, the subject to be administered, the target disease and its severity, age, sex weight, individual differences and disease state. Dosage may be repeated several times a day. Preferred is a bacterium for use, such as *Klebsiella oxytoca* for use according to the present invention, wherein said use is in a (single) dosage of between 10¹² and 10⁷ CFU, preferably between 10¹⁰ and 10⁸ CFU of said *Klebsiella oxytoca.* Doses can be applied several times, as needed.

The prevention and/or treatment of said antibiotic resistant *Enterobacteriaceae* and/or said reestablishing colonization resistance after antibiotic dysbiosis can be in the context of bacterial infection, bloodstream infection, allogeneic hematopoietic stem cell transplantation, and bacterial invasion into the liver, spleen and/or mesenteric lymph nodes (MLN) in said animal, in particular mammalian subject.

According to the present invention, the animal, in particular the mammalian subject to be treated can be preferably selected from poultry, such as chicken or geese, a mouse, rat, cat, dog, rabbit, goat, sheep, horse, camel, lama, cow, monkey, a farm animal, a sport animal, and a pet, and a human.

After establishing different animal models, the inventors isolated promising candidates for a competition with MDR *K. pneumoniae* strains. As direct competition for environmental niches in the gut is a fundamental requirement for invading species to successfully establish in the host, which is facilitated after antibiotic perturbation of the resident flora, the inventors aimed at identifying commensal bacterial species as candidates to compete with MDR *K. pneumoniae* strains. These strains would also exploit similar nutrient sources and grow under an oxidative environment. To identify and isolate commensal *Klebsiella* strains, feces of 59 healthy adults and 54 healthy children were screened and assessed in an in vitro growth assay for colonization ability with MDR *K. pneumoniae* strains to identify particularly resistant individuals.

In total, 16 *Klebsiella* species were isolated from 13 healthy adult individuals and 16 species from 15 healthy children. From 32 *Klebsiella* isolates in total, 11 strains belonged to the species *K. pneumoniae,* one strain each was identified as *K. quasipneumoniae* and *K. variicola* and 19 strains belonged to *K. oxytoca.*

In order to analyze how many of the isolated strains carry major resistance genes, genomes were sequenced by Illumina sequencing, and resistance genes were used to predict resistance phenotypes. Genomic sequencing revealed predicted resistance pattern for ampicillin, ceftriaxone, fosfomycin, and chloramphenicol. Another strain belonged to the ST23 which has been also reported as an emerging MDR strain carrying a rare hybrid plasmid harboring virulence gene (Shen D, Ma G, Li C, et al. Emergence of a multidrug-resistant hypervirulent Klebsiella pneumoniae sequence type 23 strain with a rare blaCTX-M-24-harboring virulence plasmid. Antimicrob Agents Chemother. 2019;63(3). doi:10.1128/AAC.02273-18). Two strains belonged to ST86 which was reported in 2015 as a newly emerging HV strain with serotype K2 causing life-threatening infections (Zhang Y, Sun J, Mi C, et al. First report of two rapid-onset fatal infections caused by a newly emerging hypervirulent K. Pneumonia ST86 strain of serotype K2 in China. Front Microbiol. 2015;6(JUL). doi:10.3389/fmicb.2015.00721). It became obvious how widespread resistance in *K. pneumoniae* already is in the healthy populations. Since furthermore resistance is mostly plasmid encoded it can be easily transmitted between different strains. Because of this, commensal *K. pneumoniae* strains were regarded as no good candidates to fight MDR *K. pneumoniae.*

Interestingly, in both cohorts the inventors found that occurrence of *Klebsiella* species was associated with reduced CFUs with particularly resistant individuals carrying strains of commensal *K. oxytoca.* In total, 19 strains were isolated from healthy individuals, with 4 (21.1%) strains found in healthy adults and 15 (78.9%) strains isolated from healthy children. Two to ten percent of humans harbor *K. oxytoca* in their intestines (Herzog KAT, Schneditz G, Leitner E, et al. Genotypes of Klebsiella oxytoca isolates from patients with nosocomial pneumonia are distinct from those of isolates from patients with antibiotic-associated hemorrhagic colitis. J Clin Microbiol. 2014;52(5):1607-1616. doi:10.1128/JCM.03373-13). Normally, *K, oxytoca* has been reported as an opportunistic pathogen implicated in various clinical diseases especially in infants (Chen Y, Brook TC, Soe CZ, et al. Preterm infants harbour diverse Klebsiella populations, including atypical species that encode and produce an array of antimicrobial resistance- and virulence-associated factors. Microb Genomics. May 2020:mgen000377. doi:10.1099/mgen.0.000377; Darby A, Lertpiriyapong K, Sarkar U, et al. Cytotoxic and Pathogenic Properties of Klebsiella oxytoca Isolated from Laboratory Animals. Bereswill S, ed. PLoS One. 2014;9(7):e100542. doi:10.1371/journal.pone.0100542; Reyman M, van Houten MA, van Baarle D, et al. Impact of delivery mode-associated gut microbiota dynamics on health in the first year of life. Nat Commun. 2019;10(1):4997. doi:10.1038/s41467-019-13014-7). In recent years, *K. oxytoca* has gained more clinical significance through acquisition of multiple resistances (Singh L, Cariappa MP, Kaur M. Klebsiella oxytoca: An emerging pathogen? Med J Armed Forces India. 2016;72(Suppl 1):S59-S61. doi:10.1016/j.mjafi.2016.05.002).

On the other hand, more recent studies highlighted the potential of certain *Klebsiella* strains to act as a probiotic commensal. The ability of mice to retain or share the commensal *K. michiganensis,* a closely related species to *K. oxytoca* has been shown to sufficiently maintain colonization resistance after treatment with antibiotics against an *E. coli* by competition for essential nutrients (Oliveira, R.A., Ng, K.M., Correia, M.B. et al. Klebsiella michiganensis transmission enhances resistance to Enterobacteriaceae gut invasion by nutrition competition. Nat Microbiol 5, 630-641 (2020). https://doi.org/10.1038/s41564-019-0658-4). The inventors found this species also in the most protected sample, and none of the strains exhibited further antibiotic resistance genes besides ampicillin indicating that MDR is far less widespread compared to K. *pneumoniae* isolates. Similar to *E. coli,* also some *K. oxytoca* strains may exhibit probiotic properties while other strains of the same species such as EHEC or EPEC are known pathogens (Fang K, Jin X, Hong SH. Probiotic Escherichia coli inhibits biofilm formation of pathogenic E. coli via extracellular activity of DegP. Sci Rep. 2018;8(1):1-12. doi:10.1038/s41598-018-23180-1). *K. oxytoca* isolated from protected individuals was capable to reduce growth of MDR *K.pneumoniae in vitro* and *in vivo* significantly, when added to ampicillin treated cecal content of mice before challenge with MDR *K. pneumoniae* or to Oligo-MM¹² mice without antibiotic intervention. The effect was strongly and reproducible in all animals, when reaching a certain threshold of precolonization.

The effect was present in all tested human isolates of *K. oxytoca,* and not present in a human commensal *E. coli* isolate indicating that the effect is generally present in *K. oxytoca* but is not common in other related *Enterobacteriaceae.*

In addition, the protective properties of *K. oxytoca* were shown in another MDR *K. pneumoniae* strain belonging to ST258 who is an emerging strain in the US but also worldwide, suggesting broad spectrum activity against a range of emerging *K. pneumoniae* strains. Lastly, *K. oxytoca* significantly reduced luminal and tissue associated *K. pneumoniae* in all gastrointestinal organs and inhibited systemic spread into liver and spleen in organ burden experiments.

Microbiota analysis revealed that antibiotic treated, *K. oxytoca* precolonized mice recovered their anaerobic species and species richness faster than those without *K. oxytoca.* Especially the reoccurrence of SCFA promoting anaerobic species within the *Firmicutes* and *Bacteroidetes* phylum was significantly accelerated, which was accompanied by elevated SCFA levels already after 6 days after MDR colonization.

As the protective effect is also visible in Oligo-MM¹² mice, there is the potential of each of these twelve bacteria to establish long-term protection together with *K. oxytoca* by single transfer of bacteria into GF mice.

The therapeutic potential was further supported by experiments showing the ability of *K. oxytoca* to lower the CFUs and to improve clearance of mice that were initially colonized with MDR *K. pneumoniae.* In addition, *K. oxytoca* was also capable to improve clearance in mice that were humanized with susceptible donor microbiota to the same levels of mice who received more resistant donor feces. These findings support the therapeutic or prophylactic administration of a commensal bacterium to individuals with compromised microbiota composition, which reduces both inter-patient transmission and intra-patient dissemination of highly antibiotic-resistant pathogens.

The present invention will now be described further in the following examples with reference to the accompanying Figures, nevertheless, without being limited thereto. For the purposes of the present invention, all references as cited herein are incorporated by reference in their entireties.
Figure 1 shows a diagram of recovered CFUs of *K. pneumoniae* MDR1 in differentially treated SPF-H mice cecal content. Mean and SEM of two independent experiments with n=4-5 mice per group. p values indicated represent a non-parametric Kruskal-Wallis test *p<0.05, **p<0.01, ***p<0.001, ****p<0.0001.
Figure 2 shows a diagram of the colonization kinetics of CFUs of *K. pneumoniae* MDR1 in differentially treated SPF-H mice. Mean and SEM of three independent experiments with n=4-6 mice per group.
Figure 3 shows a diagram of the colonization kinetics of CFUs of *K. pneumoniae* MDR2 in differentially treated SPF-H mice. Mean and SEM of one independent experiments with n=5 mice per group.
Figure 4 shows a diagram of the clearance rates of *K. pneumoniae* MDR1 in precolonized and control animals. P values indicated represent a log-rank test ****p<0.0001 (control/E.coli 103 vs *K. oxytoca* MK01), ####p<0.0001 (control/E.coli 103 vs *K. oxytoca* MK02).
Figure 5 shows a diagram of the clearance rates of *K. pneumoniae* MDR1 according to the examples, below. Mean of two independent experiments with n = 5 mice per group. P values indicated represent a log-rank test **p<0.01 (control vs. *K. oxytoca* MK01), #p<0.05 (control vs *K. oxytoca* MK02).
Figure 6 shows that the protective effect of commensal *K. oxytoca* is widespread among different *K. oxytoca* strains. Mean of one independent experiment with n= 3-5 mice per group. SUS = susceptible, INT = intermediate, and RES = protected donors.
Figure 7 shows the clearance rates of another *K. pneumoniae* strain (MDR2 ST258) in *K. oxytoca* precolonized and control miceMean and SEM of one out of two independent experiments with n=5 mice per group. p value indicated represent a log- rank test **p<0.01.

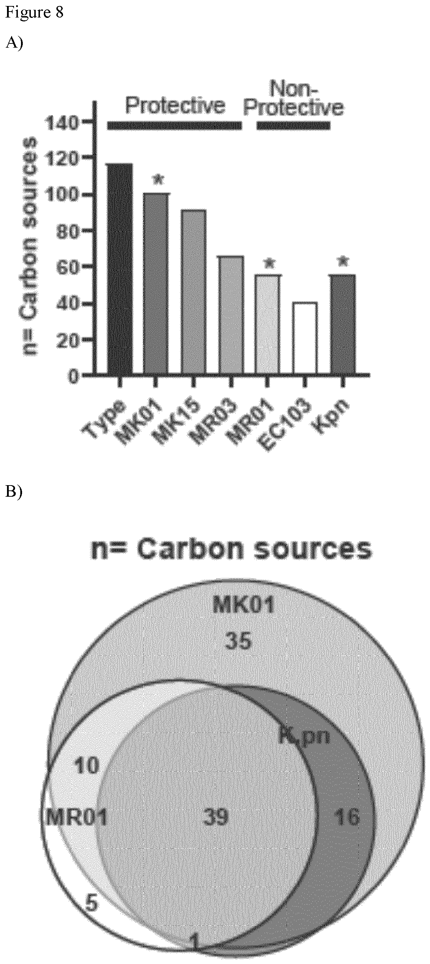

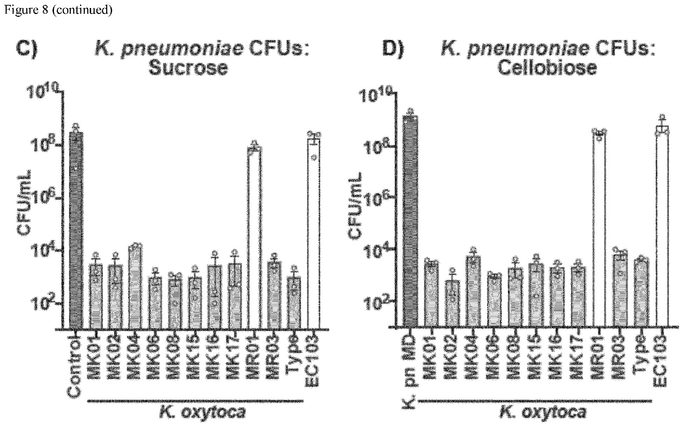

Figure 8 shows that *K. oxytoca* and *K. pneumoniae* compete for specific carbon sources with a broader substrate range found in protective *K. oxytoca* isolates. (A) Amount of carbon source with detected growth for different *K. oxytoca* isolates, *K. pneumoniae* MDR1 and *E.coli* 103. Positive growth was defined as OD > 0.2 after 24h of incubation. Strains highlighted with * were used to generate the Venn diagram displayed in (B). (C-D) CFUs of *K. pneumoniae* MDR1 after 24h of aerobic cocultivation with selected *K. oxytoca* or *E. coli* strains in minimal medium supplemented with Sucrose and Cellobiose as sole carbon sources. Values represent one out of two independent experiments with n=3 replicates per condition.

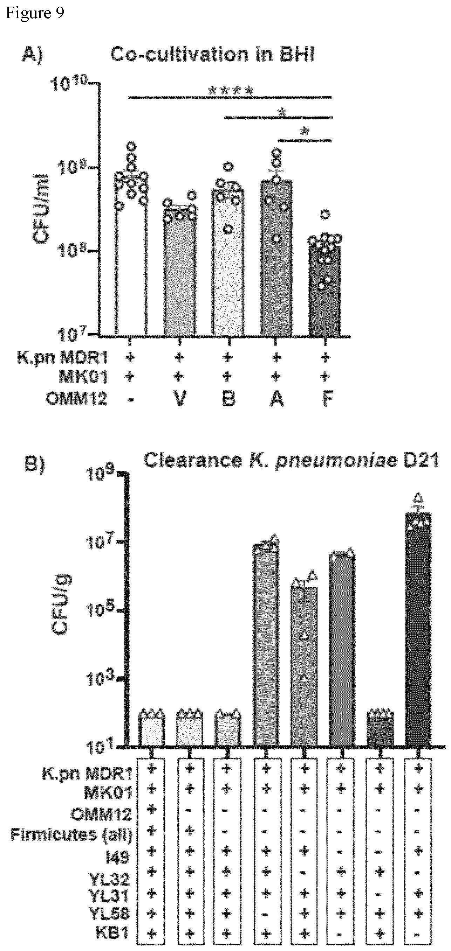

Figure 9 shows that *K. oxytoca* requires cooperating bacteria from the phylum Firmicutes to reestablish long-term colonization resistance in germfree mice. (A) Resulting CFUs of *K. pneumoniae* MDR1 after anaerobic co-cultivation with selected Oligo-MM¹² bacteria (V= Verrucomicrobia, B= Bacteroidetes, A = Actinobacteria and F = Firmicutes) and *K.oxytoca* MK01 after 24 h. Mean and SEM of three independent experiments with n=2-4 samples per group. p values indicated represent a non-parametric Kruskal-Wallis test *p<0.05, ****p<0.0001. (B) CFUs of *K. pneumoniae* MDR1 in native Oligo-MM¹² and differentially precolonized GF animals at day 21. Mice received selected Firmicutes from the Oligo-MM¹² microbiota two weeks before challenge with *K. pneumoniae* MDR1. At day -7 mice were additionally precolonized with MK01. CFUs were assessed until day 28 after *K. pneumoniae* MDR1 colonization. Table below indicates present bacteria in each group. Mean and SEM of one experiments with n=2-5 samples per group.

### EXAMPLES

### Methods

### Isolation of commensal Klebsiella strains from human stool samples

Stool samples were homogenized in 1 ml PBS and plated in serial dilutions on Simmons Citrate Agar plates supplemented with inositol, tryptophan and bile salts (SCITB) (Simmons Citrate Agar; Cheng VCC, Yam WC, Tsang LL, et al. Epidemiology of Klebsiella oxytoca-associated diarrhea detected by Simmons citrate agar supplemented with inositol, tryptophan, and bile salts. J Clin Microbiol. 2012;50(5):1571-1579. doi:10.1128/JCM.00163-12). Yellow, inositol fermenting colonies were picked and plated on LB-amp plates to check for ampicillin resistance. Positive colonies were screened with species specific PCRs for the *pehX* gene of *K. oxytoca* (Kovtunovych G, Lytvynenko T, Negrutska V, Lar O, Brisse S, Kozyrovska N. Identification of Klebsiella oxytoca using a specific PCR assay targeting the polygalacturonase pehX gene. Res Microbiol. 2003;154(8):587-592. doi:10.1016/S0923-2508(03)00148-7) or the internal transcribed spacer (ITS) of the rrn operon for *K. pneumoniae* (Liu Y, Liu C, Zheng W, et al. PCR detection of Klebsiella pneumoniae in infant formula based on 16S-23 S internal transcribed spacer. Int J Food Microbiol. 2008;125(3):230-235. doi:10.1016/j.ijfoodmicro.2008.03.005).

### In vivo K. pneumoniae colonization

If required, mice received ampicillin (0.5 g/L) for six consecutive days starting three days before colonization with *K. pneumoniae.* Inoculi were prepared by culturing bacteria overnight at 37°C in LB broth with 50 µg/ml ampicillin and 25 µg/ml cloramphenicol. Subsequently, the culture was diluted 1:25 in fresh medium, and subcultured for 4 hours at 37°C in LB broth. Bacteria were resuspended in 10 ml phosphate-buffered saline (PBS) and required amounts were calculated. Mice were orally inoculated with 10⁸ CFU of *K. pneumoniae* diluted in 200 µl PBS. Weight of the mice was monitored, and feces were collected at different time points (1, 3, 6, 9, 14, 21, 28 and 42 days) after colonization for measuring pathogen burden and 16S rRNA sequencing or mice were sacrificed at day 6 to evaluate pathogen burden in the intestinal organs.

### In vivo commensal colonization

Ampicillin pretreatment and preparation of inoculi was performed as described for *K. pneumoniae.* Mice were orally inoculated with 10⁸ CFU of commensal bacteria *(K. oxytoca* or *E. coli)* diluted in 200 µl PBS. Weight of the mice was monitored, and feces were collected at different time points (1, 3, 6, 9, 14 days) after colonization for measuring bacterial numbers and 16S rRNA sequencing.

### Quantification of fecal K. pneumoniae colonization

Fresh fecal samples were collected, and weight was recorded. Subsequently, fecal samples were diluted in 1 ml PBS and homogenized by bead-beating with 1 mm zirconia/silica beads for two times 25 seconds using a Mini-Beadbeater-96 (BioSpec). To determine CFUs, serial dilutions of homogenized samples were plated on LB plates with 50 µg/ml ampicillin and 25 µg/ml cloramphenicol. Plates were cultured at 37°C for 1 day before counting. CFUs of *K. pneumoniae* were calculated after normalization to the weight of feces. Quantification of *K. oxytoca* and *E. coli* was performed accordingly and samples were plated on LB plates with 50 µg/ml ampicillin.

### In vitro assays

Mice were sacrificed and cecal content was isolated, weighted and diluted in a 1:1 ratio with PBS or BHI and homogenized for two times 25 seconds using a Mini-BeadBeater-96 (BioSpec). Samples were either prepared aerobically or anaerobically in an anaerobic chamber. Bacteria were grown in appropriate media and normalized to 10⁶ CFUs. Tubes were inoculated with 10 µl *K. pneumoniae* (OD 0.2) and cultivated at 37°C under aerobic or anaerobic conditions for 24 hours. 25 µl of each sample was serial-diluted in 96 well plates and plated on selective agar plates with ampicillin and chloramphenicol to recover viable amounts of *K. pneumoniae.*

### Antibiotics susceptibility testing

For assessment of minimal inhibitory concentrations, the commercial ETEST kit from the vendor Biomerieux was used. Antibiotic susceptibility testing against four lead antibiotics classes was performed according to manufacturer's instructions.

### Statistical analysis

Experimental results were analyzed for statistical significance using GraphPad Prism v8.2 (GraphPad Software Inc.). Differences were analyzed by Student's t test and one-way ANOVA. P values indicated were calculated by a non-parametric Mann-Whitney U test or Kruskal-Wallis test comparison of totals between groups (Segata et al. 2011). OTUs with Kruskal-Wallis test <0.05 were considered for analysis. *P* values lower than 0.05 were considered as significant: *p<0.05, **p<0.01, ***p<0.001, ****p<0.0001.

### Mouse model for experiments

### Pretreatment with ampicillin destroys natural colonization resistance in SPF-H mice and establishes a long term colonization of the pathogen

As a basis for the experiments, a recently published gut colonization model for *K. pneumoniae* in mice (Sorbara MT, Dubin K, Littmann ER, et al. Inhibiting antibiotic-resistant Enterobacteriaceae by microbiota-mediated intracellular acidification. J Exp Med. 2018;216(1):84-98. doi:10.1084/jem.20181639) was used. First, it was verified that this animal model was also suitable to model long term colonization of MDR K. pneumoniae in SPF.

In-house SPF mice with in-house gut flora (SPF-H) were treated for 6 consecutive days with ampicillin and colonized on day 3 of treatment with 10⁸ CFUs *K. pneumoniae* MDR1, a clinical isolate belonging to ST395 previously described.

Ampicillin treated mice showed a significant higher pathogen burden especially at the beginning with CFUs reaching up to 10⁹ CFUs/ml compared to 10³ to 10⁴ CFUs/ml in control mice. In addition, these mice were unable to clear *K. pneumoniae* MDR1 even after 6 weeks of colonization suggesting a stable long-term integration of K. pneumoniae MDR1 in the microbiome of the amp-treated mice.

Weight loss or any sign of infection was absent in both groups, indicating that K. pneumoniae is an asymptomatic colonizer in healthy mice. Next, changes in the microbiota during the six weeks of *K. pneumoniae* MDR1 colonization were assessed. Regarding species richness, no changes could be observed in the microbiota of control mice, whereas richness of the amp-treated animals was almost reduced to zero after 1 day post colonization (p.c.). After the end of amp treatment, species richness recovered over time almost reaching levels similar to control animals after six weeks. Nevertheless, species evenness in these animals was still lower after 6 weeks of colonization, indicating that some long-term effects due to the antibiotic treatment were still detectable.

Therefore, the mouse colonization model established a stable long-term colonization in healthy mice without body weight loss or other signs of infection, making it a useful model to study the impact of specific commensal regarding their potential to prevent or decolonize MDR *K. pneumoniae* from the dysbiotic mouse gut and to re-establish homeostatic microbiome conditions.

The Oligo-Mouse-Microbiota (Oligo-MM¹²) is a community of 12 mouse intestinal bacteria to be used for microbiome research in gnotobiotic mice, and consists of *Clostridium innocuum, Bacteroides caecimuris, Lactobacillus reuteri, Enterococcus faecalis, Acutalibacter muris, Bifidobacterium animalis* subsp. *animalis, Muribaculum intestinale,* Flavonifractor plautii, *Clostridium clostridioforme, Akkermansia muciniphila, Turicimonas muris,* and *Blautia coccoides* (Garzetti D, Brugiroux S, Bunk B, et al. High-Quality Whole-Genome Sequences of the Oligo-Mouse-Microbiota Bacterial Community. Genome Announc. 2017;5(42):e00758-17. Published 2017 Oct 19. doi:10.1128/genomeA.00758-17).

### The ampicillin mouse model induces reproducible and robust colonization with different Klebsiella pneumoniae strains

In order to test the suitability of the mouse model with other relevant *Klebsiella* strains, the above SPF-H mice were colonized with another MDR strain from the highly relevant ST258 (*K. pneumoniae* MDR2) which is a major spread in the US This strain achieved similar CFUs as the initially tested *K. pneumoniae* MDR1. None of the mice cleared any of the strains tested even after six weeks of colonization indicating that the ampicillin mouse model works for *Klebsiella* strains in general. Similarly to what was observed with K. *pneumoniae* MDR1, mice did not lose any weight for the other strains tested indicating that immunocompetent mice did not suffer from infection with those strains.

### Screening of human stool samples for commensal Klebsiella strains - high prevalence of K. oxytoca in protected stool samples

Different age groups of healthy volunteers were screened for commensal *Klebsiella* strains. In total, 32 *Klebsiella* strains could be isolated from 29 healthy individuals, 16 species from 13 healthy adult volunteers and 16 species from 15 healthy children.

In order to analyze spread of antibiotic resistance in the *Klebsiella* isolates, genomes were sequenced using Illumina sequencing and analyzed regarding resistance genes to predict the resistance phenotype.

As expected, all of the isolates were resistant against ampicillin, confirming the occurrence of natural resistance against the antibiotics class of penicillins in *Klebsiella* (i.e. the naturally occurring ampicillin/amoxicillin-resistance phenotype). All isolated *K. oxytoca* strains did not exhibit any further resistance genes, whereas all other commensal *K. pneumoniae* strains showed at least one additional resistance with some of the strains belonging to ST395 showing even multi-drug resistance. Microbiological resistance testing verified the bioinformatics prediction. Therefore, *K. oxytoca* exhibited far less wide-spread antibiotic resistance as commensal *K. pneumoniae* strains. This further supports the concept of the present invention to use *K oxytoca* strains to fight MDR *K. pneumoniae,* since transfer of resistance apparently is less common but metabolic and ecologic behavior is similar.

### Addition of K. oxytoca to amp treated cecal content efficiently reduces CFUs of recovered K. pneumoniae in vitro

*In vitro,* SPF-H mice were treated with ampicillin for six consecutive days to diminish their natural colonization resistance. One day before sacrifice, mice were treated with *K. oxytoca* strain isolated from donor MK1903 (*K. oxytoca* MK01), MK1901 (*K. oxytoca* MK02) or left untreated as a control. Pre-colonization was assessed the next day by plating the isolated cecal content on selective agar plates. Cecal content was spiked with 10⁶ CFUs of *K. pneumoniae* MDR1 and incubated for 24h under anaerobic conditions before content was plated on LB-chloramp plates to recover viable amounts of *K. pneumoniae* MDR1. All animals were successfully colonized with *K. oxytoca.*

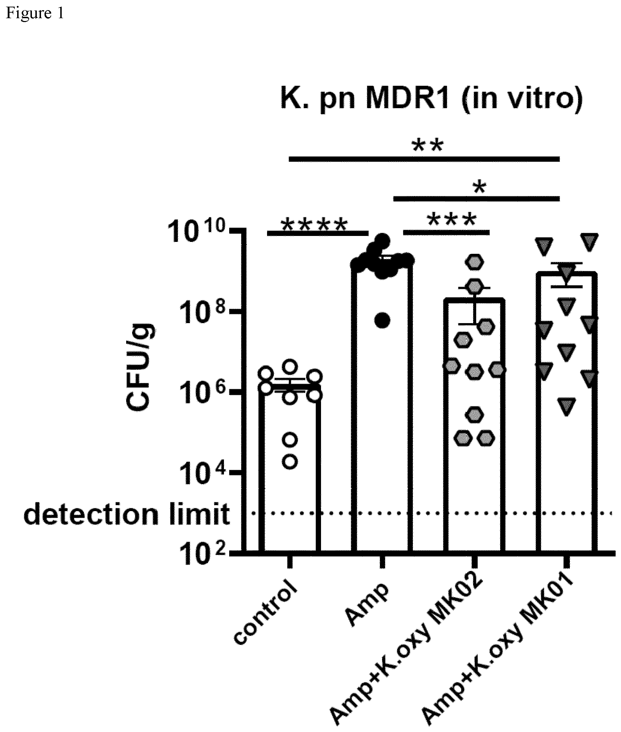

Ampicillin treatment eradicates most microbes from the SPF-H flora resulting in a significant increase of pH value in all treated groups compared to the untreated control group, which was not affected by the pre-colonization with *K. oxytoca.* Strikingly, recovered CFUs *o*f *K*. *pneumoniae* MDR1 were significantly reduced upon pre-colonization with *K. oxytoca* (Fig 1). A strong anti-correlation between the level of pre-colonization with *K. oxytoca* and the resulting CFUs of MDR *K. pneumoniae* with a coefficient of determination R² = 0.59 for *K. oxytoca* MK01 and R² = 0.68 for *K. oxytoca* MK02 was observed. *K. oxytoca* therefore has the potential to reduce MDR *K. pneumoniae.*

### Pretreatment of antibiotic-treated mice with probiotic K. oxytoca facilitates clearance of MDR K. pneumoniae from the feces

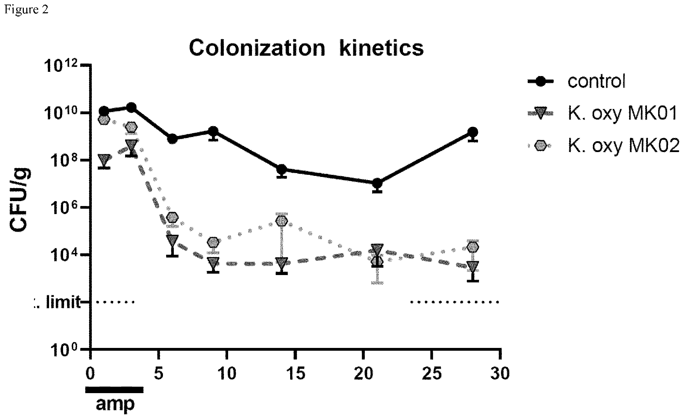

Next, it was tested whether *K, oxytoca* could also help to clear MDR *K. pneumoniae* in antibiotic disturbed microbiota situations *in vivo.* SPF-H mice were precolonized with *K. oxytoca* MK01 or *K. oxytoca* MK02 for two weeks before colonization with 10⁸ CFUs of *K. pneumoniae* MDR1. Mice received ampicillin for 6 consecutive days during each intervention three days before until three days after colonization. Feces were taken after different time points of precolonization and colonization in order to assess levels of *K. oxytoca* and MDR *K. pneumoniae.* None of the mice lost body weight upon colonization with *K. oxytoca* or MDR *K. pneumoniae.* Colonization kinetics revealed that both groups colonized with *K. oxytoca* strains exhibit significantly reduced levels of *K. pneumoniae* MDR1 (Fig. 2).

*K. oxytoca* pretreated animals could clear MDR *K.* pneumoniae faster and up to 80% (13/16 animals for *K. oxytoca* MK01 and 12/15 animals for *K. oxytoca* MK02) cleared the colonization after 28 days, whereas control animals could only clear the pathogen in 21.4 % of the cases (3/14 animals). This experiment demonstrates that *K. oxytoca* efficiently reduces fecal CFUs of MDR *K. pneumoniae* and supports clearance from the microbiome of colonized animals.

### K. oxytoca facilitates clearance of another K. pneumoniae strain from the feces

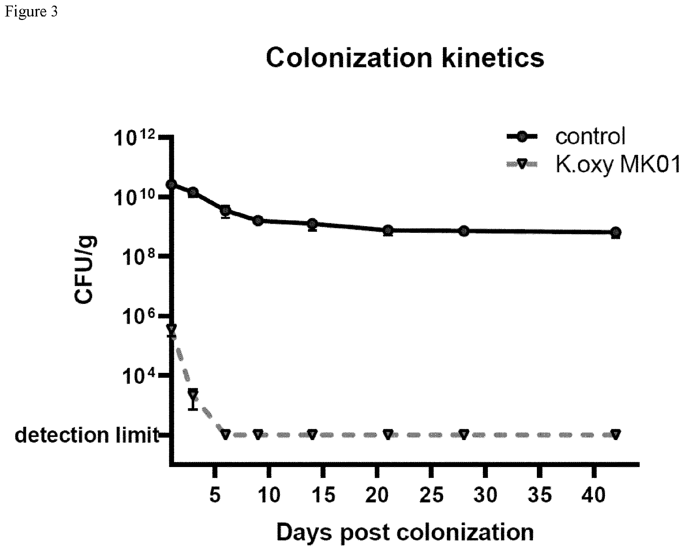

To investigate if the effect of *K. oxytoca* would also lead to the same level of protection against another MDR *K. pneumoniae* strains, ampicillin treated mice were colonized with *K*. *oxytoca* A or left uncolonized as a control. After two weeks, mice were colonized with *K. pneumoniae* MDR2, and fecal colonization was assessed after different time points of colonization. *K. oxytoca* MK01 efficiently reduced fecal CFUs of infected mice already by day 1 by a factor of 10,000 (Fig. 3). After 6 days, precolonized mice completely cleared *K*. *pneumoniae* MDR2 from the feces, resulting in a clearance rate of 100 %, which was even more efficient than initially observed for *K*. *pneumoniae* MDR1 with rates above 80%. These experiments revealed that human *K. oxytoca* strains exhibit a specific effect which is not observed with other commensal related enterobacteria, such as *E. coli,* and showed a broad-spectrum activity against different MDR *K. pneumoniae* strains.

In addition, the question was addressed whether *K. oxytoca* could also prevent colonization of MDR *K. pneumoniae* in gnotobiotic and GF mice which are naturally susceptible to colonization. Oligo-MM¹² mice which harbor 12 specific bacterial strains and no other gamma-proteobacteria as well as GF mice, which are devoid of any bacteria in the gut, were colonized with *K. oxytoca* MK01, *K. oxytoca* MK02 or *E. coli* 103 two weeks prior colonization with MDR *K. pneumoniae,* and fecal colonization was monitored over 28 days. Similar effects could be observed as initially seen in ampicillin treated SPF-H mice.

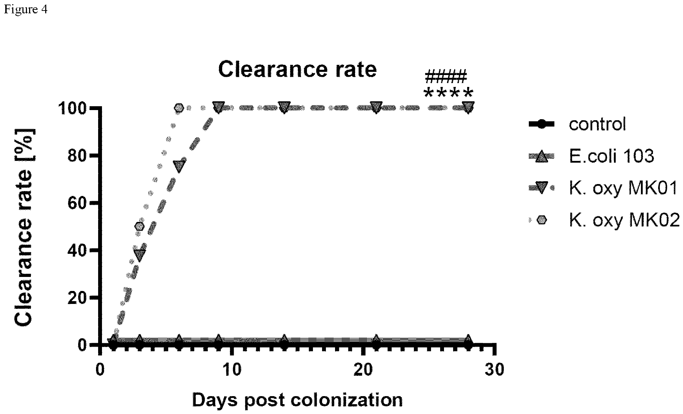

Both *K. oxytoca* strains efficiently reduced fecal CFUs of MDR K. pneumoniae and achieved total clearance after 6 *(K. oxytoca* MK02) or 9 days *(K. oxytoca* MK01) post colonization. In contrast, control group and *E. coli* 103 colonized mice exhibited similar high amount of *K. pneumoniae* during the whole course of colonization without any reduction or clearance (Fig. 4). This experiment highlighted that *K. oxytoca* is also capable to protect gnotobiotic mice with a defined microbiome composition from colonization with MDR *K. pneumoniae* indicating that the protection might occur via a direct competitive mechanism between the related *Klebsiella* species.

To further test that *K. oxytoca* actively reduces *K. pneumoniae,* GF animals were colonized with *K. oxytoca* MK01 and E. coli 103 prior to colonization with *K. pneumoniae* or mice were left untreated. Body weight loss and fecal burden was assessed over 4 weeks. All colonized mice gained up to 20% of weight during the course of experiment indicating that colonization with other enterobacteria supported the proper development of the animals. In terms of fecal colonization with MDR *K. pneumoniae* significantly reduced CFUs in the *K. oxytoca* MK01 treated animals were found, and to a lower extend also in the *E*. coli 103 colonized mice. This experiment shows that *K. oxytoca* directly inhibits the growth of MDR *K. pneumoniae,* at least in the beginning of the colonization.

### Probiotic K. oxytoca protects susceptible animals from luminal and tissue invasion and systemic dissemination of the pathogen

To examine the protective effect further, the characterization of the colonization dynamics was extended. To test if observed differences in the fecal colonization are also visible in the intestinal organs, SPF-H mice were precolonized with *K. oxytoca* MK01, *K. oxytoca* MK02 or left uncolonized as a control prior to colonization with *K. pneumoniae* MDR1. Luminal and tissue invasion of *K. pneumoniae* MDR1 was assessed in the gastrointestinal organs including small intestine, cecum and colon as well as in the liver, and lymphatic organs including spleen and mesenteric lymph nodes (MLN) on day 6 p.c. A strong reduction in the gastrointestinal organ content as well as in the tissues in both precolonized groups compared to the control groups was observed. In addition, *K. oxytoca* colonized groups did not have any bacteria in the liver, spleen or MLN in contrast to the control animals, suggesting a systemic spread of the pathogen in control mice, elevating the risk for blood stream infections. This experiment demonstrated a broad-spectrum activity of *K. oxytoca* in all gastrointestinal organs including reduction of pathogenic bacteria in the lumen and tissue and preventing the systemic spread into the liver and lymphatic tissues such as spleen and MLN.

### The adaptive immune-system is dispensable for the observed phenotype supporting the hypothesis of a direct competitive effect

To exclude the hypothesis that adaptive immunity plays a major role in the protective phenotype, the same organ burden experiment using Rag2^{-/-} SPF-H animals was performed, which are devoid of any B- and T-cell populations. The data were highly similar with the phenotype initially overserved in WT SPF-H mice. Similarly, CFUs in the gastrointestinal organs including cecum, colon and small intestine were significantly reduced in the lumen and the tissue in *K. oxytoca* A precolonized animals. In addition, bacterial burden in the liver and spleen was significantly reduced in precolonized mice indicating that *K. oxytoca* MK01 could prevent the systemic spread of the pathogen without a sufficient adaptive immune response. This supports a direct mechanism of the commensal itself rather than an immune-mediated protection.

### K. oxytoca helps anaerobic SCFA producers to recover faster after antibiotic dysbiosis thereby reestablishing colonization resistance.

In view of the results regarding an immune-mediated mechanism it was analyzed how *K*. *oxytoca* colonization may changes the microbiome composition in the mice. To do so, cecal samples from the organ burden (day 6 p.c.) were sequenced. In addition, cecal SCFA levels of these mice were determined. A strong clustering regarding the beta-diversity in precolonized animal on day 6 p.c, in the cecal content was observed. In terms of alpha diversity *K. oxytoca* treated animals displayed significantly higher species richness and evenness, and a highly diverse microbiome was observed in *K. oxytoca* precolonized animals in comparison to control animals which showed a dysbiotic species composition mainly consisting of *Klebsiella* in the phylum of *Proteobacteria,* and *Enterococcaceae* in the phylum of *Firmicutes.* Surprisingly, the microbiome of *K. oxytoca* treated animals had almost recovered full species diversity as found in untreated SPF-H mice with regard to species present and species richness. Furthermore, significantly elevated levels of butyrate and propionate in SPF-H WT and Rag2^{-/-} were found in the mice, further supporting that *K*. *oxytoca* helps to reestablish colonization resistance by facilitating the recovery of anaerobic SCFA producers. Based on these findings, SCFA concentrations can also serve as a biomarker to predict disease outcome after infection with certain *Enterobacteriaceae,* like *K. pneumoniae.*

### K. oxytoca has the therapeutic potential to cure already established MDR K. pneumoniae colonization

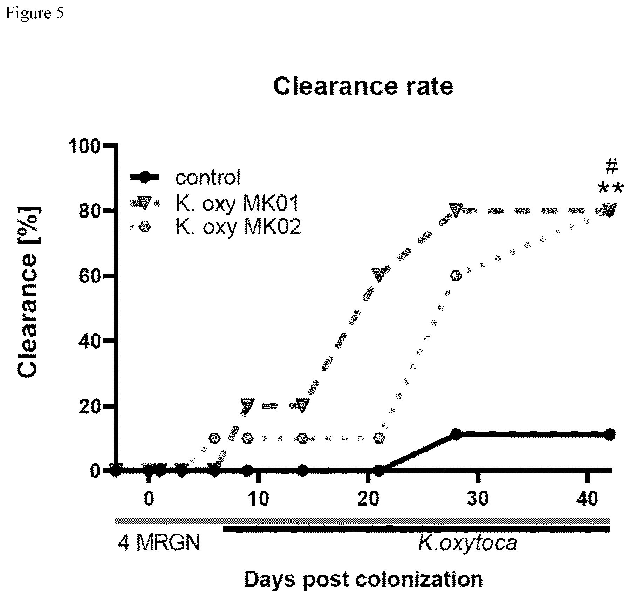

As it was shown that *K. oxytoca* has probiotic potential by efficiently blocking colonization and permanent establishment of MDR *K. pneumoniae* in the microbiome of antibiotic-treated and gnotobiotic animals, additional tests were performed, whether *K. oxytoca* could also lower or replace MDR *K. pneumoniae* after an already established colonization. To do so, mice were treated with ampicillin and infected with *K. pneumoniae* MDR1. After three days of colonization, mice were subsequently colonized with *K. oxytoca* MK01 or *K. oxytoca* MK02, and fecal burden of *K. pneumoniae* MDR1 was assessed after different time points. Mice treated with *K. oxytoca* MK01 or *K. oxytoca* MK02 cleared the colonization with *K. pneumoniae* MDR1 significantly faster when compared with the control mice (Fig 5).

After 42 days both strains *K. oxytoca* A or *K. oxytoca* B were reaching similar levels as high as 80% (8/10) clearance, whereas only one animal (11.11%) of the control group could clear the colonization (Fig. 5). In addition, to verify if the mice also cleared *K. oxytoca* from the microbiome or if the bacterium was still detectable, feces were plated on amp plates. In the *K. oxytoca* MK01 group, 5 out of 10 mice (50%) cleared the bacterium, and 3/10 (30%) cleared *K. oxytoca* MK02. This observation indicated that in most of the cases *K. pneumoniae* MDR1 was successfully replaced by *K. oxytoca.*

Taken together, this experiment supports the potential of *K. oxytoca* to act as both a potential probiotic strain and having therapeutic potential to cure MDR carriers who are already colonized with MDR *K. pneumoniae* strains.

### Protective effect of commensal K. oxytoca is strain-specific but observed against another MDR K. pneumoniae strain from the ST258

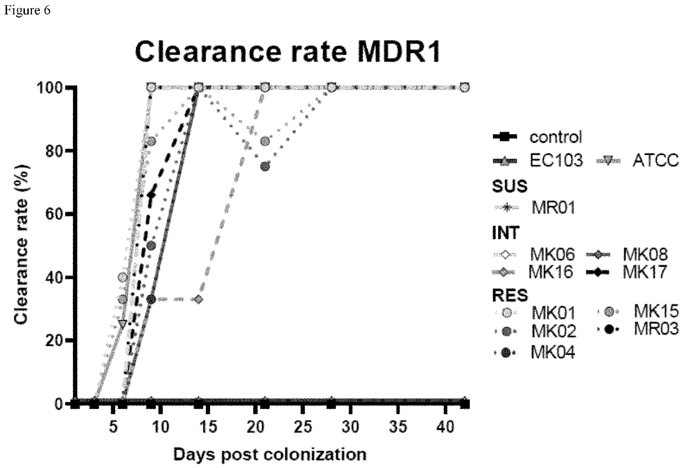

To address the question whether the protective effect is limited to a specific strain of commensal *K. oxytoca* or is shared between multiple commensal isolates or related enterobacteria in general, SPF-H were precolonized with different commensal *K. oxytoca* strains or a commensal ampicillin-resistant *E. coli* strain isolated from a healthy volunteer (strain EC103). The *K. oxytoca* strain panel included next to MK01 additional isolates from protected donors (strain MK02, MK04, MK15, MR03), intermediate donors (strain MK06, MK08, MK16, MK17) and the only isolate from a susceptible donor (MR01), as well as the type strain (ATCC13182) (Fig. 6). Importantly, no differences were observed in mice in the colonization levels between *K. oxytoca* strains and *E. coli.* Strikingly, mice precolonized with all human *K. oxytoca* isolates except for MR01 promoted decolonization of *K. pneumoniae* MDR1 similar compared to MK01 (Fig. 6). In contrast, neither *K. oxytoca* MR01 nor *E*. *coli* EC103 efficiently promoted *K. pneumoniae* MDR1 decolonization.

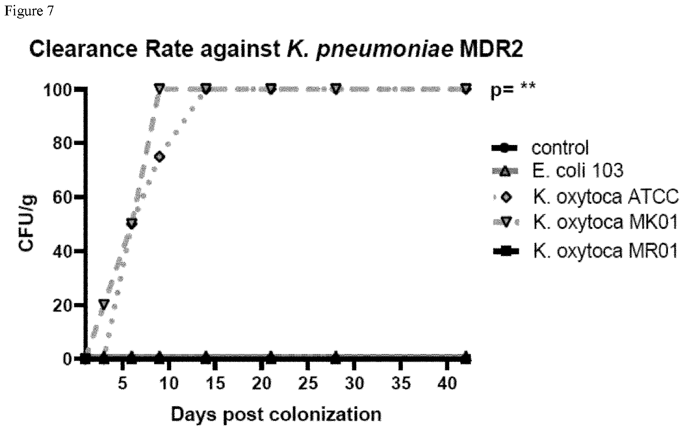

To investigate if the protective effects of different *K. oxytoca* strains are also observed against other *K. pneumoniae* strains, mice were precolonized with either *K. oxytoca* strains (MK01, MR01, ATCC13182) and *E. coli* (EC103) and then colonized with *K. pneumoniae* strain MDR2 (ST258) (Fig. 7). In line with the previous observations MK01 and ATCC efficiently reduced fecal colonization of MDR2 already by day 1 by a factor of 10,000, whereas no decolonization could be observed for MR01 and *E. coli.* Strikingly, MK01-precolonized mice completely cleared *K. pneumoniae* MDR2 within 9 days from the feces. Taken together, these experiments revealed that all tested commensal *K. oxytoca* strains except for MR01 promoted efficient clearance of *K. pneumoniae* strains from at least two different sequence types.

### K. oxytoca and K. pneumoniae compete in the gut lumen for specific carbon sources with a broader substrate range found in protective K. oxytoca isolates

The inventors tested whether both bacteria *K. oxytoca* and *K. pneumoniae* occupy similar spatial locations in the gut. FISH analysis of the gut lumen demonstrated that both Klebsiella species appeared to be distributed within the lumen, suggesting that they share similar spatial niches and therefore compete for nutrients. To test *in vitro,* whether both *Klebsiella* strains coexist or compete for the same substrates in the mouse gut, cecal content of germfree mice was spiked with different *K. oxytoca* strains or *E. coli* as control and *K. pneumoniae* and incubated for 24h under anaerobic conditions. Cocultivation of all commensal *K. oxytoca* strains except for MR01 and EC103 significantly reduced CFUs *of K. pneumoniae* MDR1 *in vitro* by a factor of 100-1000 fold. Protective *K. oxytoca* isolates were able to utilize more different carbon sources compared to *K. pneumoniae* and the non-protective strains (Fig. 8). For instance, MK01 could utilize 100 carbon sources whereas MR01 could only utilize in total 55 different carbon sources. In comparison to *K. pneumoniae,* which could use 56 different carbon sources, MK01 but not MR01 covered with an overlap of 55 carbon sources almost the complete carbon source utilization pattern of *K. pneumoniae* except for lactulose. Similar patterns were observed for the other protective strains indicating that protective *K*. *oxytoca* strains have a broader substrate range covering the substrate range of *K pneumoniae.*

### Protective K. oxytoca strains outcompete K. pneumoniae for non-aromatic beta-glycosides utilization

Focussing on sugars that could be utilized by *K. pneumoniae* MDR1 and protective strains, 13 differences between the panel of protective (n=4) and non-protective (n=2) strains in the utilization capacity of various monosaccharides (Tagatose, Psicose, beta-Methyl-D-Glucoside), disaccharides (Cellobiose, Gentiobiose, Palatinose, Sucrose), oligosaccharides (Stachyose), aryl-beta-Glycosides (Arbutin, Salicin) and sugar alcohols (Dulcitol, Inositol, D-Arabitol) were identified (Fig 8). Interestingly, 6 out of 13 candidates from different groups shared the structural similarity having a beta-glucosidic bond (beta-Methyl-D-Glucoside, Cellobiose, Gentiobiose, Sucrose, Arbutin and Salicin) indicating that beta-glucoside utilization contributes to interspecies competition of *Klebsiella* strains. Phenotypic screening revealed that all protective *K. oxytoca* strains (n=22) but not MR01 and EC103 could utilize aryl-beta glucosides (Arbutin and Salicin) and other beta-glucosidic sugars (Cellobiose and Sucrose) as a sole carbon source in minimal medium. In order to test whether these *K. oxytoca* strains could indeed outcompete *K. pneumoniae* for beta-Glucosides utilization, both species were co-cultured in a 1:1 ratio in minimal medium supplemented with Sucrose, Cellobiose, Salicin or Arbutin as sole carbon sources for 24h under anaerobic conditions. Selective plating revealed that growth of *K. pneumoniae* in Sucrose and Cellobiose supplemented minimal medium was reduced 1,000 to 10,000-fold in co-cultures with all *K. oxytoca* strains as tested except for MR01 or as a control with EC103 (Fig 8). Notably, no inhibition was observed in minimal medium supplemented with Salicin and Arbutin. Thus, *K. oxytoca* effectively outcompetes *K. pneumoniae in vitro* for non-arylic beta-glucosides like sucrose and cellobiose but not for aromatic beta-glucosides like arbutin and salicin or sugar alcohols like dulcitol or inositol.

### In order to maintain long-term full colonization resistance in germfree mice, K. oxytoca requires additional bacteria

To characterize whether *K. oxytoca* directly outcompetes *K. pneumoniae* in the gut environment, germfree (GF) mice were precolonized with protective *K. oxytoca* MK01, or as controls with the non-protective MR01 and EC103 and then challenged with *K*. *pneumoniae* MDR1 (Fig 9). At the early timepoint of colonization, *K. oxytoca* MK01 but not MR01 reduced *K. pneumoniae* MDR1 colonization compared to GF mice with 1000-fold reduction of *K. pneumoniae* CFUs at day 3 p.c.. Yet, this reduction slowly decreased but remained stable until 42 days with still significantly reduced CFUs detected in *K. oxytoca* MK01-precolonized mice in comparison to control mice. Of note, 25% of *K. oxytoca* MK01-precolonized GF mice cleared *K. pneumoniae* MDR1 compared to 80-100% in *K. oxytoca* MK01-precolonized SPF-H mice, suggesting that direct metabolic competition is a major driver for protection in the initial phase of luminal expansion but cooperating bacteria are required to achieve long-term decolonization.

*K. oxytoca* MK01 efficiently reduced *K. pneumoniae* MDR1 colonization resulting in gut decolonization already within 9 days. In contrast, *E. coli* EC103 and MR01 colonized Oligo-MM¹² mice exhibited similarly high colonization levels of *K. pneumoniae* MDR1 as control mice.

Colonization of germfree mice with various different minimal consortia of Firmicutes bacteria from the Oligo-MM¹² microbiota revealed that a minimum of three additional bacteria namely *Blautia coccoides* YL58, *Enterococcus faecalis* KB1 and [*Clostridium*] *clostridioforme* YL32 were able together with *K. oxytoca* to achieve long-term clearance of *K. pneumoniae* MDR1 (Fig 9). This demonstrates that specific members of the phylum *Firmicutes* cooperate with *K. oxytoca* to promote decolonization of *K. pneumoniae* from the gut, with the cooperating bacteria presumably further restricting the availability of alternative carbon sources.

## Claims

1. A bacterium selected from the group of *Klebsiella oxytoca* and species related to *Klebsiella oxytoca* for use in the prevention and/or treatment of antibiotic resistant *Enterobacteriaceae,* such as *Klebsiella pneumoniae,* in particular multi-resistant *Enterobacteriaceae,* in the microbiome of an animal, in particular a mammalian subject, wherein said bacterium selected from the group of *Klebsiella oxytoca* and species related to *Klebsiella oxytoca* outcompetes said *Enterobacteriaceae* for use of beta-glucosidic sugars such as sucrose and/or cellobiose.

2. Bacterium for use according to claim 1, wherein said prevention and/or treatment is through decolonization of said *Enterobacteriaceae.*

3. Bacterium for use according to claim 1 or 2, wherein said species related to *Klebsiella oxytoca* is selected from the group consisting of *K. michiganensis, K. grimontii,* K. *aerogenes,* and *Klebsiella* that have a carbohydrate utilization pattern substantially similar to *K. oxytoca* strain MK01.

4. Bacterium for use according to any one of claims 1 to 3 in reestablishing colonization resistance after antibiotic dysbiosis in the microbiome of an animal, in particular a mammalian subject.

5. Bacterium for use according to any one of claims 1 to 4, wherein said microbiome is located in the gut of said animal, in particular said mammalian subject.

6. Bacterium for use according to any one of claims 1 to 5, wherein said bacterium for use is a *Klebsiella* strain isolated from a human.

7. Bacterium for use according to any one of claims 1 to 6, wherein said bacterium for use does not exhibit additional antibiotic-resistance in addition to the naturally occurring ampicillin/amoxicillin-resistance phenotype.

8. Bacterium for use according to any one of claims 1 to 7, wherein said prevention and/or treatment of said antibiotic resistant *Enterobacteriaceae* and/or said reestablishing colonization resistance after antibiotic dysbiosis is in the context of bacterial infection, bloodstream infection, allogeneic hematopoietic stem cell transplantation, and bacterial invasion into the liver, spleen and/or mesenteric lymph nodes (MLN) in said animal, in particular said mammalian subject.

9. Bacterium for use according to any one of claims 1 to 8, wherein said use is through fecal microbiota transplant (FMT), in a suitable medium, such as sterile saline, or in a suitable solid dosage form, such as a suppository or capsule, in particular stomach-acid resistant, or as a probiotic.

10. Bacterium for use according to any one of claims 1 to 9, wherein said use is in a dosage of between 10¹² and 10⁷ CFU, preferably between 10¹⁰ and 10⁸ CFU of said bacterium.

11. Bacterium for use according to any one of claims 1 to 10, wherein said use is in combination with at least one bacterial species selected from the group consisting of *Clostridium* spec., in particular *Clostridium innocuum* and *Clostridium clostridioforme. Bacteroides* spec., in particular *Bacteroides caecimuris, Lactobacillus* spec., in particular *Lactobacillus reuteri, Enterococcus* spec., in particular *Enterococcus faecalis, Acutalibacter* spec., in particular *Acutalibacter muris, Bifidobacterium* spec., in particular *Bifidobacterium animalis* subsp. *animalis, Muribaculum* spec., in particular *Muribaculum intestinale, Flavonifractor* spec., in particular *Flavonifractor plautii, Akkermansia* spec., in particular *Akkermansia muciniphila, Turicimonas* spec., in particular *Turicimonas muris,* and *Blautia* spec., in particular *Blautia coccoides,* Firmicutes spec., and preferably *Blautia coccoides* YL58, *Enterococcus faecalis* KB1 and *Clostridium clostridioforme* YL32, and in particular human isolates thereof.

12. A method for preventing and/or treating antibiotic resistant *Enterobacteriaceae,* such as *Klebsiella pneumoniae,* in the microbiome of an animal, in particular a mammalian subject, such as a human, comprising administering to said animal, in particular said mammalian subject an effective amount of a bacterium selected from the group of *Klebsiella oxytoca* and species related to *Klebsiella oxytoca,* wherein said bacterium selected from the group of *Klebsiella oxytoca* and species related to *Klebsiella oxytoca* outcompetes said *Enterobacteriaceae* for use of beta-glucosidic sugars such as sucrose and/or cellobiose, and wherein preferably said related species is selected from the group consisting of *K. michiganensis, K. grimontii,* K. *aerogenes,* and *Klebsiella* that have a carbohydrate utilization pattern substantially similar to *K. oxytoca* strain MK01.

13. The method according to claim 12, wherein said preventing and/or treating is in combination with at least one bacterial species selected from the group consisting of *Clostridium* spec., in particular *Clostridium innocuum* and *Clostridium clostridioforme, Bacteroides* spec., in particular *Bacteroides caecimuris, Lactobacillus* spec., in particular *Lactobacillus reuteri, Enterococcus* spec., in particular *Enterococcus faecalis, Acutalibacter* spec., in particular *Acutalibacter muris, Bifidobacterium* spec., in particular *Bifidobacterium animalis* subsp. *animalis, Muribaculum* spec., in particular *Muribaculum intestinale, Flavonifractor* spec., in particular *Flavonifractor plautii, Akkermansia* spec., in particular *Akkermansia muciniphila, Turicimonas* spec., in particular *Turicimonas muris,* and *Blautia* spec., in particular *Blautia coccoides,* Firmicutes spec., and preferably *Blautia coccoides* YL58, *Enterococcus faecalis* KB 1 and *Clostridium clostridioforme* YL32, and in particular human isolates thereof.
